# EUROPEAN PATENT SPECIFICATION

(11) **EP 3 576 549 B1**
(45) Date of publication and mention of the grant of the patent: **13.08.2025**
(21) Application number: 18707983.5
(22) Date of filing: 01.02.2018
(51) Int. Cl.: A61K 31/718, A23L 33/00, A23L 33/135, A23L 33/17, A23L 33/175, A23L 33/21, A23L 2/39, A61K 38/05, A61K 38/06, A61K 31/198, A61K 31/702, A61K 35/745

(54) **ACCELERATION OF LEARNING ABILITY AND/OR ACCELERATION OF REACHING MEMORY OBJECTIVES IN PRETERM OR INFANT BORN SMALL FOR GESTATIONAL AGE BY NUTRITONAL COMPOSITIONS COMPRISING PREBIOTIC FIBER, PROBIOTIC BACTERIUM AND GLUTAMINE**
BESCHLEUNIGUNG DER LERNFÄHIGKEIT UND/ODER BESCHLEUNIGUNG DES ERREICHENS VON GEDÄCHTNISZIELEN BEI FRÜHGEBORENEN ODER SÄUGLINGEN, DIE FÜR DAS SCHWANGERSCHAFTSALTER KLEIN GEBOREN WERDEN, DURCH NAHRUNGSZUSAMMENSETZUNGEN, DIE PRÄBIOTISCHE FASER, PROBIOTISCHES BAKTERIUM UND GLUTAMIN UMFASSEN
ACCÉLÉRATION DE LA CAPACITÉ D'APPRENTISSAGE ET/OU ACCÉLÉRATION DE L'ATTEINTE DES OBJECTIFS DE MÉMOIRE CHEZ LES NOURRISSONS PRÉMATURÉS OU LES NOURRISSONS NÉS PETITS POUR L'ÂGE GESTATIONNEL PAR DES COMPOSITIONS NUTRITIONNELLES COMPRENANT UNE FIBRE PRÉBIOTIQUE, UNE BACTÉRIE PROBIOTIQUE ET DE LA GLUTAMINE

(30) Priority: 01.02.2017 NL 1050065
(43) Date of publication of application: 11.12.2019
(73) Proprietor: N.V. Nutricia, 2712 HM Zoetermeer (NL)
(72) Inventor: RENES, Ingrid Brunhilde, 3584 CT Utrecht (NL); VAN ELBURG, Ruurd, 3584 CT Utrecht (NL); BARTKE, Nana, 3584 CT Utrecht (NL)
(74) Representative: Nederlandsch Octrooibureau
(86) International application number: PCT/NL2018/050072
(87) International publication number: WO 2018/143806

(56) References cited:
- WO-A1-2013/105851
- US-A1- 2015 024 082
- US-A1- 2015 366 919
- DATABASE EMBASE [online] ELSEVIER SCIENCE PUBLISHERS, AMSTERDAM, NL; February 2010 (2010-02-01), HIKARU U ET AL: "Bifidobacteria prevents preterm infants from developing infection and sepsis", XP002770660, Database accession no. EMB-2010589688
- INTERNATIONAL JOURNAL OF PROBIOTICS AND PREBIOTICS 2010 NEW CENTURY HEALTH PUBLISHERS, LLC USA, vol. 5, no. 1, February 2010 (2010-02-01), pages 33 - 36, ISSN: 1555-1431
- KRISTIN KEUNEN ET AL: "Impact of nutrition on brain development and its neuroprotective implications following preterm birth", PEDIATRIC RESEARCH, vol. 77, no. 1-2, 14 October 2014 (2014-10-14), US, pages 148 - 155, XP055376883, ISSN: 0031-3998, DOI: 10.1038/pr.2014.171

## Description

### FIELD OF THE INVENTION

The present invention relates to nutritional compositions comprising synbiotics and glutamine for accelerating the maturation of brain structure and accelerating the ability of learning and maturation of cognition. The invention is in particular suitable for infants that are born prematurely.

### BACKGROUND OF THE INVENTION

Increasing attention is raised towards the role of nutrition early in life on the development of the brain and possibilities to modulate brain functioning. A complex, bidirectional communication system exists between the gut and the brain, which ensures gastrointestinal homeostasis and digestion maintenance, and which in reverse direction may affect cognitive function. Especially in infants an imbalance is unwanted since during infancy the cognitive system is still developing, and the disturbance of this development may have long lasting effects.

Breast-feeding is the preferred method of feeding infants. However, there are circumstances that make breast-feeding impossible or less desirable. In those cases infant formulae are a good alternative. The composition of modern infant formulae is adapted in such a way that it meets many of the special nutritional requirements of the fast growing and developing infant. Still further improvements can be made. The present invention relates to nutritional compositions for infants, in particular infant formulae, which comprises specific ingredients for modulating brain structure and functioning.

In a review, Keunen et al, in Pediatric Research, 2015, vol 77(1) discuss a role of glutamine and of probiotics and of prebiotic fibers in nutrition on brain development and its neuroprotective implications following preterm birth.

WO2014/070016 discloses that dietary supplementation with *Bifidobacterium breve* in combination with non-digestible oligosaccharides, preferably further in combination with glutamine, resulted in improved cognitive and behavioral performance and in particular that anxiety levels were lowered and spatial memory was improved.

US 2015/024082 discloses compositions enriched in glutamine to improve structural brain development in preterm and/or very low birth weight (VLBW) infants. The compositions also include prebiotics.

WO 2013/105851 discloses that supplementation of preterm formula with glutamine and prebiotic improves brain microstructure development. Furthermore, the compositions improve neurocognitive functioning, behavioral and/or motional development.

### SUMMARY OF THE INVENTION

The present inventors now surprisingly found in an animal model study that supplementation of the diet with a prebiotic and a probiotic and glutamine, in brain tissue, microstructural maturation was enhanced evidenced by beneficial influences on orientation dispersion index in brain cortical gray matter, white matter fiber bundles radial diffusivity and fractional anisotropy values.

In addition it was surprisingly found that the test animals receiving a diet supplemented with a prebiotic and a probiotic and glutamine, were quicker in learning the objective set out in a T-maze compared to the control group.

In view of these findings, the use of a prebiotic and a probiotic and glutamine is particularly suitable to enhance maturation of brain microstructure in mammalian offspring, in particular in an infant.

Moreover, in view of the present findings, the use of a prebiotic and a probiotic and glutamine is particularly suitable to accelerate maturation of cognition, accelerate learning ability and/or accelerate reaching memory objectives in mammalian offspring, in particular in an infant.

### DETAILED DESCRIPTION OF THE INVENTION

The invention concerns a nutritional composition comprising a prebiotic fiber and a probiotic bacterium and glutamine, for use in accelerating learning ability and/or accelerating reaching memory objectives in an infant, wherein the infant is a preterm infant and/or an infant that is born small for gestational age (SGA), and wherein the acceleration is in comparison to preterm infants and/or infants that are born small for gestational age (SGA) not being adminstered the nutritional composition comprising prebiotic fiber and probiotic bacterium and glutamine.

### Prebiotic fiber

In the composition for use according to the present invention, the nutritional composition comprises a prebiotic fiber. The term prebiotic fiber is known in the art and refers to dietary fibers or non-digestible saccharides which are nutritional components that are typically resistant to digestion and absorption in the small intestine, hence which are not or only partially digested by the action of acids or digestive enzymes such as present in for instance small intestine and stomach, with preferably a complete or partial fermentation in the large intestine, in particular by intestinal flora. For example, sucrose, lactose, maltose and maltodextrins are considered digestible. For example, galacto-oligosaccharides, fructo-oligosaccharides and fructo-polysaccharides are considered non-digestible saccharide. Thus preferably according to the present invention, the term 'prebiotic fiber' refers to non-digestible saccharide.

The term "saccharide" as used in the present context preferably refers to a saccharide with a degree of polymerization (DP) of 2 to 250, preferably a DP of 2 to 100, more preferably of 2 to 60. It is understood that in the context of this invention a saccharide with a DP in a certain range may include a mixture of saccharides with different average DP's, for example, if a saccharide with a DP of 2 to 100 is included in the present composition, this may include compositions which contain oligosaccharides with an average DP between 2 and 5, and polysaccharides with an average DP between 20 and 60. In the context of the present invention, the term 'oligosaccharide' refers to a prebiotic fiber with an average DP below 10 and the term 'polysaccharide' refers to a prebiotic fiber with an average DP of 10 and higher.

The nutritional composition for the use indicated in claim 1 preferably comprises a prebiotic, or non-digestible saccharide, selected from the group consisting fructo-oligosaccharide, galacto-oligosaccharide, fructo-polysaccharide, gluco-oligosaccharide, arabino-oligosaccharide, mannan-oligosaccharide, xylo-oligosaccharide, fuco-oligosaccharide, arabinogalacto-oligosaccharide, glucomanno-oligosaccharide, galactomanno-oligosaccharide, arabino-polysaccharide, mannan-polysaccharide, xylo-polysaccharide, fuco-polysaccharide, arabinogalacto-polysaccharide, glucomanno-polysaccharide, galactomanno-polysaccharide, sialic acid comprising oligosaccharide, sialic acid comprising polysaccharide, uronic acid oligosaccharide and uronic acid ploysaccharide.

The nutritional composition for the use indicated in claim 1 preferably comprises a prebiotic fiber selected from the group consisting of a fructo-oligosaccharide, a galacto-oligosaccharide, a fructo-polysaccharide, a combination of a fructo-oligosaccharide and a galacto-oligosaccharide tide, a combination of a fructo-oligosaccharide and a fructo-polysaccharide, a combination of a galacto-oligosaccharide and a fructo-polysaccharide and a combination of a fructo-oligosaccharide, a galacto-oligosaccharide and a fructo-polysaccharide.

Preferably the nutritional composition for the use indicated in claim 1 comprises galacto-oligosaccharide, more preferably transgalacto-oligosaccharide. Preferably the nutritional composition comprises galacto-oligosaccharide with an average DP between 2 and 10.

In one embodiment preferably the nutritional composition for the use indicated in claim 1 comprises fructo-polysaccharide. Preferably the nutritional composition comprises fructo-polysaccharide with an average DP between 10 and 60, preferably with an average DP between 20 and 60.

In a preferred embodiment the nutritional composition comprises a mixture of galacto-oligosaccharide and fructo-polysaccharide. Preferably the nutritional composition comprises galacto-oligosaccharide with an average DP between 2 and 10 and fructo-polysaccharide with an average DP between 10 and 60, preferably with an average DP between 20 and 60. Preferably the nutritional composition comprises galacto-oligosaccharides and fructo-polysaccharides in a weight ratio of 20 to 0.5, more preferably 20 to 1, most preferably from 12 to 2.

In one embodiment preferably the nutritional composition for the use indicated in claim 1 comprises fructo-oligosaccharide. Preferably the nutritional composition comprises fructo-oligosaccharide with an average DP between 2 and 10. In a preferred embodiment the nutritional composition comprises a mixture of fructo-oligosaccharide and fructo-polysaccharide. Preferably the nutritional composition comprises fructo-oligosaccharide with an average DP between 2 and 10 and fructo-polysaccharide with an average DP between 10 and 60, preferably with an average DP between 20 and 60. Preferably the nutritional composition comprises fructo-oligosaccharides and fructo-polysaccharides in a weight ratio of 20 to 0.05, more preferably 10 to 0.1.

In one embodiment preferably the nutritional composition for the use indicated in claim 1 comprises a mixture of galacto-oligosaccharide and fructo-oligosaccharide Preferably the nutritional composition comprises galacto-oligosaccharide with an average DP between 2 and 10 and fructo-oligosaccharide with an average DP between 2 and 10. Preferably the nutritional composition comprises galacto-oligosaccharides and fructo-oligosaccharides in a weight ratio of 20 to 0.05, more preferably 10 to 0.1.

In one embodiment preferably the nutritional composition for the use indicated in claim 1 comprises a mixture of galacto-oligosaccharide and fructo-oligosaccharide and fructo-polysaccharide. Preferably the nutritional composition comprises galacto-oligosaccharide with an average DP between 2 and 10 and fructo-oligosaccharide with an average DP between 2 and 10 and fructo-polysaccharide with an average DP between 10 and 60, preferably with an average DP between 20 and 60. Preferably the nutritional composition comprises galacto-oligosaccharides and fructo-oligosaccharides and fructo-polysaccharides in a weight ratio of to 20 to 1 : 10 to 0.05 : 10 to 0.05.

In one embodiment, the nutritional composition for the use indicated in claim 1 comprises 0.5 to 20 wt.% prebiotic fiber per g dry weigth of the nutritional composition, more preferably 0.5 to 10 wt.%, even more preferably 1.5 to 7.5 wt.%. In one embodiment, the nutritional composition for the use indicated in claim 1 comprises 80 mg to 2 g prebiotic fiber per 100 ml, more preferably 150 mg to 1.50 g, even more preferably 300 mg to 1 g per 100 ml.

### Probiotic

The nutritional composition for the use indicated in claim 1 comprises a probiotic. The term probiotic is known in the art and refers to micro-organisms, preferably bacteria, which have a beneficial effect on a host when ingested by or administered to that host. In the nutritional composition for the use indicated in claim 1 the probiotic is preferably a lactic acid producing bacteria.

The nutritional composition for the use indicated in claim 1 preferably comprises a probiotic of the genus *Lactobacillus* or *Bifidobacterium* or of both. In a preferred embodiment, the *Lactobacillus* is one or more selected from *L. rhamnosus, L. casei, L. paracasei, L. helveticus, L. delbrueckii, L. reuteri, L. brevis, L. crispatus, L. sakei, L. jensenii, L. sanfransiscensis, L. fructivorans, L. kefiri, L. curvatus, L. paraplantarum, L. kefirgranum, L. parakefir, L. fermentum, L. plantarum, L. acidophilus, L. johnsonii, L. gasseri, L. xylosus, L. salivarius* etc.

Preferred species are *L. rhamnosus, L. casei, L. paracasei, L. reuteri, L. crispatus, .L fermentum L. plantarum L. acidophilus, L. johnsonii L. gasseri* and *L. salivarius,* more preferably one or more selected from *L. plantarum, L. casei* and *L. rhamnosus.,* more preferably the prociotic comprises a strain belonging to the species *L. casei.* In a preferred embodiment, the *Bifidobacterium* is one or more selected from *B. longum, B. breve, B. animalis, B. infantis, B. bifidum, B. adolescentis, B. pseudolongum, B. catenulatum, B. pseudocatenulatum, B. angulatum* more preferably *B. breve.*

The nutritional composition for the use indicated in claim 1 preferably comprises a *Bifidobacterium.* Preferably the nutritional composition comprises a *Bifidobacterium breve. Bifidobacterium breve* is a Gram-positive, anaerobic, branched rod-shaped bacterium. The *B. breve* preferably has at least 95 % identity of the 16 S rRNA sequence when compared to the type strain of *B. breve* ATCC 15700, more preferably at least 97% identity (Stackebrandt & Goebel, 1994, Int. J. Syst. Bacteriol. 44:846-849). Preferred *B. breve* strains are those isolated from the faeces of healthy human milk-fed infants. Typically, these are commercially available from producers of lactic acid bacteria, but they can also be directly isolated from faeces, identified, characterized and produced. According to a preferred embodiment, the present composition for the use indicated in claim 1 contains at least one *B. breve* selected from the group consisting of *B. breve* Bb-03 (Rhodia/Danisco), *B. breve* M-16V (Morinaga), *B. breve* R0070 (Institute Rosell, Lallemand), B. breve BR03 (Probiotical), B. breve BR92) (Cell Biotech), DSM 20091, LMG 11613, YIT4065, FERM BP-6223 and CNCM I-2219. Most preferably, the *B. breve* is selected from the group consisting of *B. breve* M-16V and *B. breve* CNCM I-2219, most preferably M-16V. *B. breve* I-2219 was published in WO 2004/093899 and was deposited at the Collection Nationale de Cultures de Microorganisms, Institute Pasteur, Paris, France on 31 May 1999 by Compagnie Gervais Danone. *B. breve* M-16V was deposited as BCCM/LMG23729 and is commercially available from Morinaga Milk Industry Co., Ltd.

The nutritional composition for the use indicated in claim 1 preferably comprises 10² to 10¹³ colony forming units (cfu) of a probiotic, preferably *B. breve,* per gram dry weight of the nutritional composition, preferably 10⁴ to 10¹², more preferably 10⁵ to 10¹⁰, most preferably from 10⁵ to 1x10⁸ cfu of a probiotic, preferably *B. breve B. breve,* per gram dry weight of the nutritional composition. The amount of the probiotic, preferably *B. breve,* in the nutritional composition for the use indicated in claim 1 is preferably administered at a daily dose of 10² to 10¹³, more preferably from 10⁵ to 10¹², most preferably from 10⁷ to 5x10⁹ colony forming units (cfu).

Preferably the nutritional composition comprises 10³ to 10¹³ cfu of a probiotic, preferably B. *breve,* per 100 ml, more preferably 10⁶ to 10¹¹ cfu per 100 ml, most preferably 10⁷ to 10⁹ cfu per 100 ml.

The nutritional composition for the use indicated in claim 1 preferably comprises viable probiotic, preferably *B. breve.* Alternatively, the nutritional composition for the use indicated in claim 1 preferably comprises non-viable probiotic, preferably *B. breve* equivalent to the amounts of cfu as described above. The equivalent of cfu can be determined by performing the 5'nuclease assay with appropriate probes and primers, preferably the *B. breve* probes and primers as disclosed in WO 2005/039319 in the nutritional composition comprising non-viable *B. breve* and compare this with a calibration curve obtained from a comparable nutritional composition to which known amounts in cfu of viable probiotic, preferably *B. breve* have been added. Viable bifidobacteria can be commercially obtained as described above. Probiotic cells, preferably B. *breve* cells can be made non-viable by methods known in the art, including heat treatment steps (including sterilization, pasteurization, UHT treatment), radiation (UV), treatment with oxygen, treatment with bactericidals such as ethanol, sonication, ultra-high pressure application, high pressure homogenization and use of a cell disruptor. Preferably the probiotic, preferably *B. breve* is heat-killed. The presence of non-viable probiotic, preferably *B. breve,* advantageously provides many product technological benefits, including increased shelf-life, a reduced incidence of bacterial contamination, decreased post-acidification of the product, improved dosage control and improved convenience of reconstitution.

### Glutamine

Glutamine in the present invention refers to L-glutamine. Glutamine is one of the most abundant amino acids in plasma and human milk and is considered conditionally essential in preterm infants. Glutamine is utilized as a source of energy and for nucleotide synthesis in all rapidly dividing cells, such as the intestinal lining and certain immune cells. In the brain, glutamine is a substrate for neurotransmitters and an important source of energy for the nervous system.

Glutamine is preferably present in an easily absorbable form. In particular in infants with an immature intestinal tract, glutamine present in intact protein is less easily absorbed. Therefore the nutritional composition for the use indicated in claim 1 according to the present invention preferably comprises glutamine in the form of free amino acid, glutamine containing dipeptide and/or glutamine containing tripeptide, most preferably glutamine containing dipeptide and/or free glutamine. Free glutamine and glutamine containing dipeptide and glutamine containing tripeptide are commercially available, for example at Ajinomoto, USA.

The nutritional composition for the use indicated in claim 1 of the present invention preferably comprises glutamine levels higher than normally present in human milk protein or standard infant formula based on cow's milk derived protein. Preferably the nutritional composition for the use indicated in claim 1 of the present invention comprises at least 12 wt.%, more preferably at least 15 wt.%, even more preferably at least 30 wt.% glutamine based on total protein. Preferably the nutritional composition comprises less than 80 wt.%, more preferably less than 50 wt.% glutamine based on total protein. Preferably the nutritional composition comprises at least 1.5 wt.%, more preferably at least 2 wt.%, even more preferably at least 4 wt.% glutamine based on dry weight of the nutritional composition. Preferably the nutritional composition comprises less than 20 wt.%, more preferably less than 10 wt.% glutamine based on dry weight of the nutritional composition. Preferably the nutritional composition for the use indicated in claim 1 of the present invention comprises at least 0.3 g, more preferably at least 0.5 g, even more preferably at least 1 g glutamine based on 100 kcal of the nutritional composition. Preferably the nutritional composition comprises less than 5 g, even more preferably less than 2 g glutamine based on 100 kcal of the nutritional composition. Preferably the nutritional composition comprises at least 0.4 g, more preferably at least 0.6 g, even more preferably at least 1.25 g glutamine per 100 ml. Preferably the nutritional composition comprises less than 6 g, even more preferably less than 2.5 g glutamine per 100 ml.

Preferably the nutritional composition comprises at least 12 wt.%, more preferably at least 15 wt.%, even more preferably at least 30 wt.% glutamine in the form of free amino acid and glutamine containing dipeptide and glutamine containing tripeptide based on total protein. Preferably the nutritional composition comprises less than 80 wt.%, more preferably less than 50 wt.% glutamine in the form of free amino acid and glutamine containing dipeptide and glutamine containing tripeptide based on total protein. Preferably the nutritional composition comprises at least 1.5 wt.%, more preferably at least 2 wt.%, even more preferably at least 4 wt.% glutamine in the form of free amino acid and glutamine containing dipeptide and glutamine containing tripeptide, based on dry weight of the nutritional composition. Preferably the nutritional composition comprises less than 20 wt.%, more preferably less than 10 wt.% glutamine in the form of free amino acid and glutamine containing dipeptide and glutamine containing tripeptide, based on dry weight of the nutritional composition. Preferably the nutritional composition comprises at least 0.3 g, more preferably at least 0.5 g, even more preferably at least 1 g glutamine in the form of free amino acid and glutamine containing dipeptide and glutamine containing tripeptide, based on 100 kcal. Preferably the nutritional composition comprises less than 5 g, even more preferably less than 2 g glutamine in the form of free amino acid and glutamine containing dipeptide and glutamine containing tripeptide, based on 100 kcal. Preferably the nutritional composition comprises at least 0.4 g, more preferably at least 0.6 g, even more preferably at least 1.25 g glutamine in the form of free amino acid and glutamine containing dipeptide and glutamine containing tripeptide per 100 ml. Preferably the nutritional composition comprises less than 6 g, even more preferably less than 2.5 g glutamine in the form of free amino acid and glutamine containing dipeptide and glutamine containing tripeptide per 100 ml.

In one embodiment the nutritional composition for the use indicated in claim 1 according to the invention is a nutritional supplement. In the present context, the nutritional supplement is also referred to as a glutamine-based supplement. Preferably the nutritional supplement, or the glutamine-based supplement is in the form of a powder, preferably in a unit dose. In a preferred embodiment the glutamine-based supplement in a unit dose, preferably in the form of a powder, comprises at least 5 wt.%, or at least 8 wt.% glutamine in the form of free glutamine, glutamine dipeptide and/or glutamine tripeptide based on dry weight of the glutamine-based supplement. Preferably the glutamine-based supplement comprises at least 30 wt.%, more preferably at least 50 wt.%, most preferably more than 75 wt.% glutamine based on total protein. Preferably the glutamine-based supplement comprises at least 1.5 wt.%, more preferably at least 2 wt.%, more preferably at least 4 wt.% glutamine, even more preferably at least 15 wt.%, even more preferably at least 40 wt.%, most preferably at least 75 wt.% glutamine based on dry weight of the glutamine-based supplement. Preferably the glutamine-based supplement comprises at least 0.3 g, more preferably at least 0.5 g, more preferably at least 1 g glutamine, even more preferably at least 5, more preferably at least 10, most preferably at least 20 g glutamine based on 100 kcal.

In a preferred embodiment, the glutamine-based supplement for the use indicated in claim 1 is added to human milk, standard preterm formula, or human milk fortified with standard human milk fortifier in such an amount that the final nutritional composition glutamine enriched nutritional composition comprises at least 12 wt.%, more preferably at least 15 wt.%, more preferably at least 20 wt.%, even more preferably at least 30 wt.% glutamine based on total protein. Preferably the nutritional supplement for the use indicated in claim 1 is added to human milk, standard preterm formula, or human milk fortified with standard human milk fortifier in such an amount that the final glutamine enriched nutritional composition comprises at least 1.5 wt.%, more preferably at least 2 wt.%, even more preferably at least 4 wt.% glutamine based on dry weight of the nutritional composition. Preferably the nutritional supplement for the use indicated in claim 1 is added to human milk, standard preterm formula, or human milk fortified with standard human milk fortifier in such an amount that the final glutamine enriched nutritional composition comprises at least 0.3 g, more preferably at least 0.5 g, even more preferably at least 1 g glutamine based on 100 kcal.

Preferably the nutritional supplement for the use indicated in claim 1 is added to human milk, standard preterm formula, or human milk fortified with standard human milk fortifier in such an amount that the final glutamine enriched nutritional composition comprises at least 12 wt.%, more preferably at least 15 wt.%, more preferably at least 20 wt.%, even more preferably at least 30 wt.% glutamine in the form of free amino acid and/or dipeptide based on total protein. Preferably the nutritional supplement for the use indicated in claim 1 is added to human milk, standard preterm formula, or human milk fortified with standard human milk fortifier in such an amount that the final glutamine enriched nutritional composition comprises at least 1.5 wt.%, more preferably at least 2 wt.%, even more preferably at least 4 wt.% glutamine in the form of amino acid and/or dipeptide based on dry weight of the nutritional composition. Preferably the nutritional supplement for the use indicated in claim 1 is added to human milk, standard preterm formula, or human milk fortified with standard human milk fortifier in such an amount that the final glutamine enriched nutritional composition comprises at least 0.3 g, more preferably at least 0.5 g, even more preferably at least 1 g glutamine in the form of free amino acid and/or dipeptide based on 100 kcal.

Preferably, the daily dose of glutamine provided to the infant is between 0.01 and 0.5 g/kg body weight, preferably between 0.05 and 0.4 g/kg body weight, even more preferably between 0.1 and 0.35 g/kg body weight.

### Nutritional composition

The nutritional composition for the use indicated in claim 1 according to the present invention advantageously concerns a composition wherein the lipid provides 5 to 50% of the total calories, the protein provides 5 to 50% of the total calories, and the carbohydrate provides 15 to 90% of the total calories. Preferably, in the nutritional composition the lipid provides 35 to 50% of the total calories, the protein provides 7.5 to 12.5% of the total calories, and the carbohydrate provides 40 to 55% of the total calories. For calculation of the % of total calories for the protein component, the total of energy provided by the proteins, peptides and amino acids needs to be taken into account.

The nutritional composition preferably comprises at least one lipid selected from the group consisting of animal lipid (excluding human lipids) and vegetable lipids. Preferably the present composition comprises a combination of vegetable lipids and at least one oil selected from the group consisting of fish oil, animal oil, algae oil, fungal oil, and bacterial oil. The nutritional composition comprising a prebiotic fiber and a probiotic excludes human milk.

The nutritional composition preferably comprises protein. The protein in the nutritional composition is preferably selected from the group consisting of non-human animal proteins (preferably milk proteins, preferably proteins from cow's milk), vegetable proteins (preferably soy protein and/or rice protein), free amino acids and mixtures thereof. The nutritional composition preferably contains casein, whey, hydrolyzed casein and/or hydrolyzed whey protein. Preferably the protein comprises intact proteins, more preferably intact bovine whey proteins and/or intact bovine casein proteins. As the present invention concerns a nutritional composition for use according to claim 1 in preterm or SGA infants, the protein is preferably selected from the group consisting of hydrolyzed milk protein, more preferably selected from the group consisting of hydrolyzed whey protein and hydrolyzed casein.

The nutritional composition preferably comprises digestible carbohydrates. The nutritional composition preferably comprises a digestible carbohydrate, wherein at least 35 wt.%, more preferably at least 50 wt.%, more preferably at least 75 wt.%, even more preferably at least 90 wt.%, most preferably at least 95 wt.% is lactose. The nutritional composition preferably comprises at least 25 grams lactose per 100 gram dry weight of the nutritional composition, preferably at least 40 grams lactose per 100 gram.

When in liquid from, the nutritional composition preferably has a caloric density between 0.1 and 2.5 kcal/ml, even more preferably a caloric density of between 0.5 and 1.5 kcal/ml, most preferably between 0.6 and 0.8 kcal/ml. The amount of nutritional composition administered per day is preferably between 50 and 2000 ml, more preferably between 200 and 1500, most preferably between 400 and 1000 ml.

Preferably the nutritional composition for the use indicated in claim 1 according to the present invention is an infant formula. In one embodiment the nutritional composition for the use indicated in claim 1 according to the present invention is a preterm infant formula, or briefly preterm formula. The preterm formula comprises all macro- and micronutrients needed for preterm infants so as to achieve a growth similar to fetal growth coupled with satisfactory functional development.

In a preferred embodiment the preterm formula comprises from 5 to 25 wt.% protein, preferably 9 to 20 wt.%, more preferably 13 to 18 wt.% protein based on the dry weight of the preterm formula. In a preferred embodiment the preterm formula comprises from 1.8 to 3.0 g protein, preferably, preferably 2.0 to 3.0 g, preferably 2.5 g to 2.6 g protein, per 100 ml.

The preterm formula in ready to drink form has in a preferred embodiment about 70 to 90 kcal, preferably 75 to 85 kcal per 100 ml. The preterm formula preferably has an osmolarity below 450 mOsmol/l, more preferably below 400, even more preferably below 350. Particularly in preterm infants, a too high osmolarity is a disadvantage.

In one embodiment, the nutritional composition for the use indicated in claim 1 according to the invention is in dry form, preferably in the form of a powder. This powder is suitable for reconstitution with water or another aqueous phase. When the nutritional composition is in powder form it advantageously has a better shelf life.

### LC-PUFA

Preferably the nutritional composition for the use indicated in claim 1 according to the present invention comprises long chain poly unsaturated fatty acids (LC-PUFA), more preferably n-3 and n-6 LC-PUFA, even more preferable arachidonic acid (ARA) and docosahexaenoic acid (DHA). LC-PUFA is an important part of the fatty acyl chain composition of the brain membranes and therefore advantageously enhances brain microstructure maturation and supports accelerating maturation of cognition, supports accelerating learning ability and/or supports accelerating reaching memory objectives. The presence of LC-PUFA, in particular ARA and DHA, will have a further improved, or even synergistic, beneficial effect together with the prebiotic fiber, the probiotic and the glutamine.

n-3 LC-PUFA, in particular docosahexaenoic acid (DHA), is an important part of the fatty acyl chain composition of the brain membranes and advantageously enhances brain microstructure maturation. More preferably, the nutritional composition comprises n-3 LC-PUFA, even more preferably DHA. Since a low concentration of DHA is already effective, the content of n-3 LC-PUFA in the nutritional composition, preferably does not exceed 15 wt.% of the total fatty acid content, preferably does not exceed 10 wt.%, even more preferably does not exceed 5 wt.%. Preferably the nutritional composition comprises at least 0.2 wt.%, preferably at least 0.5 wt.%, more preferably at least 0.75 wt.% n-3 LC-PUFA of the total fatty acid content. The DHA content preferably does not exceed 5 wt.%, more preferably does not exceed 1 wt.%, but is preferably at least 0.1 wt.% of the total fatty acid. Preferably as a source of n-3 LC-PUFA single cell oil, preferably algal oil, fungal oil and/or microbial oil is used, since these oil sources have a low EPA/DHA ratio, which results in a beneficial effect on the brain. More preferably the nutritional composition comprises fish oil, more preferably tuna oil.

n-6 LC-PUFA, in particular arachidonic acid (ARA) is an important part of the fatty acyl chain composition of the brain membranes and advantageously enhances brain microstructure maturation.. The nutritional composition preferably comprises relatively low amounts of ARA. The n-6 LC-PUFA content preferably does not exceed 5 wt.%, more preferably does not exceed 0.8 wt.%, more preferably does not exceed 0.75 wt.%, even more preferably does not exceed 0.5 wt.% based on total fatty acids. Since ARA is important in infants for optimal functional membranes, especially membranes of neurological tissues, the amount of n-6 LC-PUFA is preferably at least 0.02 wt.%, more preferably at least 0.05 wt.%, even more preferably at least 0.1 wt.% based on total fatty acids, more preferably at least 0.25 wt.%.

The weight ratio n-6 LC-PUFA / n-3 LC-PUFA, in particular the weight ratio of ARA/DHA in the nutritional composition is preferably from 3 to 0.5, more preferably from 2 to 1. Preferably the weight ratio is above 1. These ratio's ensure an optimal brain functioning.

LC-PUFA are preferably provided as free fatty acids, in triglyceride form, in diglyceride form, in monoglyceride form, in phospholipid form, or as a mixture of one of more of the above. Preferably the nutritional composition contains LC-PUFA in triglyceride and/or phospholipid form, even more preferably phospholipid form since LC-PUFA in phospholipid form are better incorporated into membranes. Therefore a dietary source of LC-PUFA in the form of phospholipids will have a further improved effect on the brain than when administered in form of triglycerides. A preferred source of LC-PUFA therefore is egg phospholipid. Commercial sources of egg oil, rich in phospholipids, and having arachidonic and docosahexaenoic fatty acyl chains in the phospholipid molecules are known.

### Application

Infants have an age of 0-12 months, toddlers have an age of 12-36 months. The nutritional composition for the use indicated in claim 1 is preferably enterally administered, more preferably orally. The present composition is preferably a nutritional formula, preferably an infant formula. The nutritional composition can advantageously be applied as a complete nutrition for infants. The nutritional composition preferably comprises lipid, protein, and carbohydrate and is preferably administered in liquid form. Dry nutritional compositions, e.g. powders, can be accompanied with instructions as to admix said dry compositions, in particular nutritional formula, with a suitable liquid, e.g. water.

The nutritional composition for the use indicated in claim 1 is for a preterm infant. A preterm infant can also be named a premature infant. A subgroup of premature infants are very preterm infants. A preterm infants is born before the end of the 37th week of pregnancy. A very preterm infant is born before the end of the 32th week of pregnancy. In one embodiment, the nutritional composition for the use indicated in claim 1 is for an infant that is born small for gestational age (SGA). An SGA infant is an infant whose birth weight lies below the 10th percentile for that gestational age. An SGA infant has usually been the subject of intrauterine growth restriction (IUGR). Premature and/or SGA infants include low birth weight infants (LBW infants), very low birth weight infants (VLBW infants), and extremely low birth weight infants (ELBW infants). LBW infants are defined as infants with a weight less than 2500 g. VLBW infants as infants with a weight which is less than 1500 g, and ELBW infants as infants with a weight less than 1000 g. Preterm infants have an immature intestinal tract, hence it is preferred that the nutritional composition according to the invention is administered to the preterm infant starting at least in the first two weeks after birth, preferably within the first week after birth, more preferably at least within 5 days after birth, even more preferably at least within 3 days after birth, most preferably at least within 2 days after birth.

The nutritional composition of claim 1 might be for use in infants born via caesarean section (said use is not necessarily according to the present invention as set forth in claim 1). A caesarean section (C-section) is a surgical procedure where an infant is delivered through an incision made in the mother's abdominal wall, and then through the wall of the uterus. It is of relevance to improve and/or accelerate developing the appropriate Bifidobacteria population and Bifidobacterium species diversity in the gastrointestinal tract of C-section delivered infants at the onset of life outside the womb, and that will contribute to create a favourable gut ecosystem milieu through their metabolic capability. Administration to the infant delivered via C-section could start at least in the first two weeks after birth, preferably within the first week after birth, more preferably at least within 5 days after birth, even more preferably at least within 3 days after birth, most preferably at least within 2 days after birth.

The present inventions concerns accelerating learning ability and/or accelerating reaching memory objectives preferably in a preterm infant.

Preferably the beneficial effects of accelerating learning ability and/or accelerating reaching memory objectives occurs in the first 24 months of life, preferably in the first 12 months of life, more preferably in the first 6 months of flife, more preferably in the first 3 months of life, most preferably in the first month of life.

Hence preferably the nutritional composition for the use indicated in claim 1 is adminstered in the first 24 months of life. However, as there are scientifically-based indications that the window of opportunity wherein the infant is more susceptible to cognition- and brain-related effects of nutritional interventions lies closer to the day of birth, the nutritional composition for the use indicated in claim 1 is preferably adminstered in the first 12 months of life, more preferably in the first 6 months of life, more preferably in the first 3 months of life, most preferably in the first month of life. In a preferred embodiment, the nutritional composition for the use indicated in claim 1 is administered to preterm infants starting at least in the first two weeks after birth, preferably within the first week after birth, more preferably at least within 5 days after birth, even more preferably at least within 3 days after birth, most preferably at least within 2 days after birth.

### EXAMPLES

### Animal procedures, experimental diets and housing

Forty piglets from two litters were delivered by cesarean section at 90% gestation and housed and handled as described previously (Andersen AD *et al.,* Delayed growth, motor function and learning in preterm pigs during early postnatal life. Am J Physiol Regul Integr Comp Physiol. 2016 Jan 13). Passive immunity was provided to all piglets by infusion of maternal plasma at 5h (4 ml/kg), 13h (5 ml/kg), and 21h (7 ml/kg) after birth. Initially, all piglets received parenteral nutrition (PN, modified Kabiven, Fresenius Kabi, Bad Homburg, Germany). PN volumes were advanced from 96-144 mL/kg/day from day 1-3. The enteral basal milk diet in both groups consisted of raw bovine milk that was fed from day 1, gradually increasing over the study period (32-224 mL/kg/d). The piglets were randomly assigned to two groups: the intervention group and control group (PPG vs CON). During day 2-23, scGOS and IcFOS were added in a ratio of 9:1 to the milk diet in the PPG group. Glutamine concentration was kept constant at 0.3 g/kg/d throughout the experiment in the PPG group. *B. breve* M16-V was provided at 3.0 x 10⁹ CFU per animal per day (reconstituted in 1 mL raw milk) as a single daily dose in the PPG group. The control group (CON) was given the same volume of 1 mL using maltodextrin (0.03 g/ml) as placebo. To match the extra carbohydrate and protein provided in the PPG diet, the CON diet was prepared by adding glucose at 4.14 g/L and lactose at 4.0 g/L.

On day 23, all piglets were DEXA-scanned in ventral recumbency following anesthesia with an intra-muscular injection of a zoletil. Subsequently, the pigs were euthanized with an intracardiac injection of sodium pentobarbital. The brain was removed from the skull and wet weights of the cerebrum, cerebellum, brain stem (including the midbrain, pons and medulla oblongata), caudate nucleus, and hippocampus were obtained. The right cerebral hemisphere and the cerebellum were immediately immersed in 4% formaldehyde and sub-regions (hippocampus, caudate nucleus, prefrontal cortex) were dissected from the left cerebral hemisphere and snap frozen.

### Example 1: Neurobehavioral assessments

Cognition was assessed in a spatial maze task designed with clear plexiglas walls as a plus-shaped maze where one of two possible start arms was sealed off to form a T-maze during testing. The maze was surrounded by gray curtains to avoid unintended shift of focus during testing and which at the same time allowed access for placing and removing individual pigs from the maze. Four posters with different colors and patterns attached to the curtains served as extra maze cues, by which the pigs had to learn to navigate and remember to obtain a milk reward (1-2 mL of cow's milk). For each pig, this accessible reward was placed in a fixed maze arm (e.g. east) while an equal amount of inaccessible milk was placed in the opposite arm (west) to mask olfactory cues. All piglets were tested for six days (10 trials / session) and the starting position in each trial within a session was altered (north or south arm) by block randomization to ensure that the starting position was balanced within a single session. The alternating positions force the pigs to solve the maze by using the visual cues and apply an allocentric learning strategy to reach the learning criterion (≥ 8 out of 10 correct choices). This performance criterion of 80% correct indicates that the piglets had successfully acquired the task. At each test session, a food-deprived pig was placed in the maze and the experimenter left the room. A trial began when a guillotine door to the start box was opened by use of a string system operated from the adjacent room. The trial ended when the pig passed a choice line marked on the floor of the east or west arm of the maze. All sessions were video recorded and subsequently analyzed with Ethovision XT10 providing information on distance travelled and latency to choice.

### Results

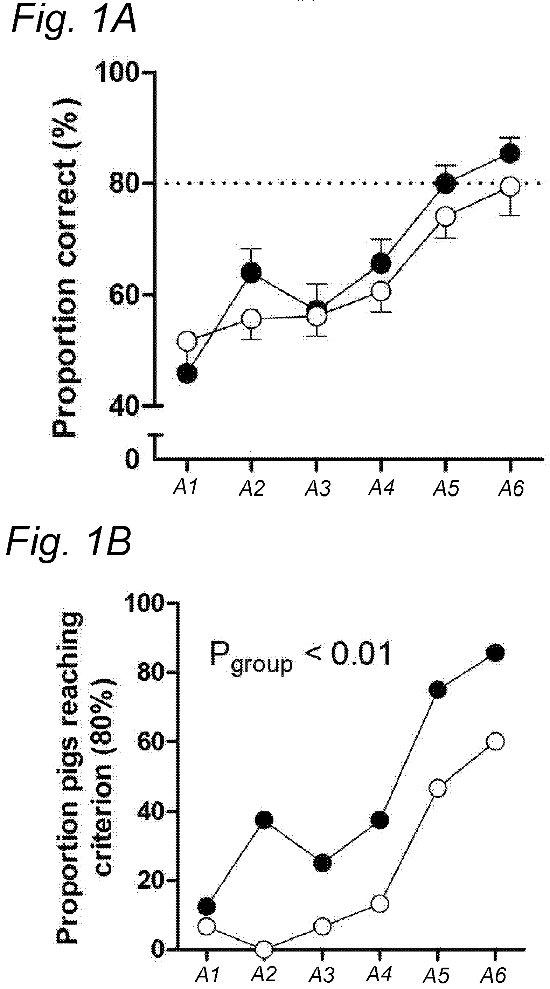

Performance in the T-maze, indicated as the mean proportion of correct choices, increased over time in both groups (P<0.001). Surprisingly, however, in this learning and memory task, the ability of PPG piglets to locate the food reward improved over time with piglets surpassing the performance criterion of 80% correct by day 5 of acquisition in an accelerated manner compared to CON animals. CON animals required 1 day more (Figure 1A).

As the average proportion of correct choices across a treatment group is influenced by pigs performing either very high or very low, a complementary way of displaying the data is the proportion of pigs reaching the learning criterion of 80% correct choices over time. From this analysis, the increase in the proportion of pigs reaching the learning criterion was higher in the PPG vs. CON pigs (P<0.01, Figure 1B).

Both groups had a similar motivation to solve the task, as evidenced from a lack of statistical differences for both speed of decision-making and distance travelled during the task between the two groups.

### Example 2: Ex vivo magnetic resonance imaging (MRI) analyses of brain microstructure (nutritional compositions for use in modification of brain microstructure are not falling under the scope of protection of the present invention)

Fixed cerebral hemispheres were subjected to diffusion tensor MR imaging. MR experiments were performed on an actively-shielded 9.4T/31 cm magnet (Agilent) equipped with 12 cm gradient coils (400 mT/m, 120 µs) with a 3.5 mm diameter birdcage coil. A multi-b-value shell protocol was acquired using a spin-echo sequence with the following parameters: FOV = 30×23 mm2, matrix size = 128×96, 20 slices of 1 mm thickness in the axial plane with a gap of 0.5 mm between each, 3 averages with TE/TR = 45/3000 ms. A total of 96 diffusion weighted images were acquired, 15 of them as b0 reference images. The remaining 81 were separated in 3 shells with the following distribution (# of directions/b-value in s/mm2): 21/1750, 30/3400 and 30/5100. All 81 directions were non-collinear and were uniformly distributed in each shell. The total acquisition time was 15h per brain. Acquired data were fitted using the NODDI toolbox (55). The diffusion tensor (DT) was spatially normalized to the study-specific DT template using DTI-TK (56). The regions of interest (ROI) were drawn on the DT study-specific template and were then transformed back to the subject space in order to compute ROI-averaged estimates of DTI and NODDI maps. Four different brain regions were identified on the DT-template: cortex (Cx), corpus callosum (CC), internal capsule (IC) and Cortico-cortical tract (CCT).

### Results

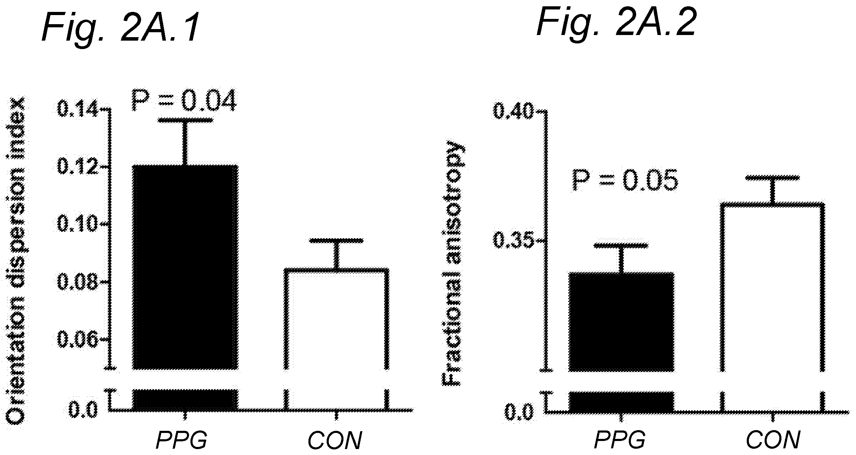

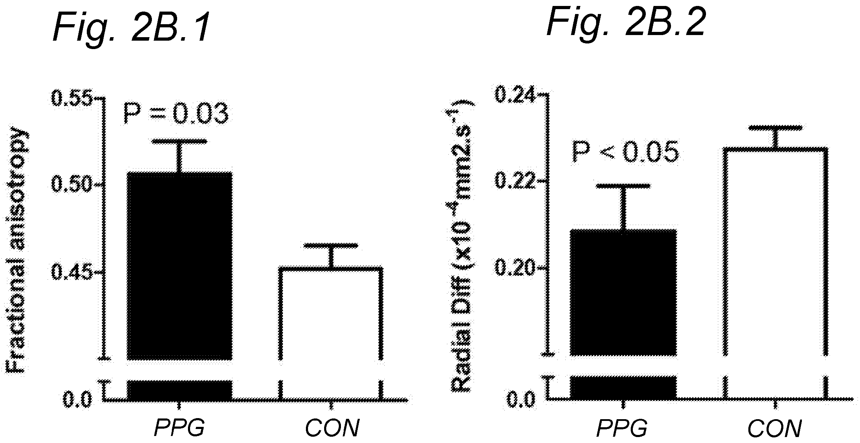

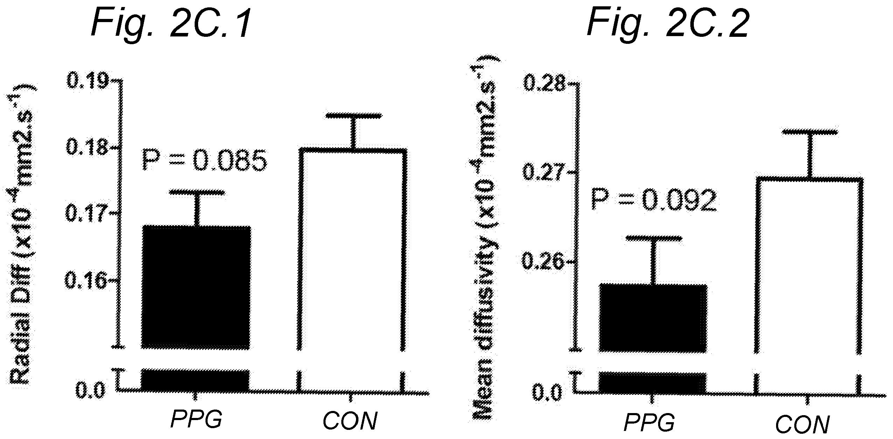

Brain white matter microstructure assessed on NODDI derived parameters of the right cerebral hemisphere, showed ~8% reduced radial diffusivity (P<0.05) and 12% increased fractional anisotropy values (P<0.05) in the cortico-cortical tract (Figure 2B), and tendencies to reduced radial (~7%) and mean (~5%) diffusivities in the internal capsule (Figure 2C; both P=0.09), in PPG pigs compared to the values in CON pigs. This is consistent with an increased maturation of association and projection white matter fiber bundles in the PPG group, respectively. In the gray matter, fractional anisotropy values were ~7% lower (P=0.06) and the orientation dispersion index (ODI) ~43% higher in PPG pigs (Figure 2A; P<0.05), both of which are consistent with advanced microstructural maturational processes in the gray matter.

### Statistics

Group differences in growth were assessed by repeated measures ANOVA adjusted for sex, birth weight and litter using the lme function in the software package R (version 3.1.3). Similarly, group mean performance, latency to choice, distance travelled within the maze and home cage activity were analyzed with repeated measures adjusted for litter and initially adjusted also for sex. The increment in proportion of pigs performing ≥ 80% in the T-maze was analyzed with repeated measures logistic regression adjusted for litter using the glmer-function.

### Example 3: Preterm formula with prebiotic fiber, B. breve ana glutamine (compositions as such not falling under the scope of the present invention)

Preterm formula in powder form comprising per 100 g about 474 kcal, 15.6 g protein, 49.8 g digestible carbohydrates (mainly lactose and maltodextrin), 22.9 g fat and 4.7 g non-digestible oligosaccharides. The protein comprises about 92.5 wt.% casein and whey protein from cow's milk based on total protein in a weight ratio of 1:1.5. About 7.5 wt.% of the protein is free L-glutamine. The non-digestible oligosaccharides are galacto-oligosaccharides (Source Vivinal GOS, Borculo Domo) and fructo-polysaccharides (Source RaftilinHP, Orafti ) in a weight ratio of 9:1. Due to the EU directives the non digestible disaccharides in the GOS do not qualify as dietary fiber. Hence the fiber content is labeled to be 3.3 g per 100 g powder. 10⁷ cfu of *Bifidobacterium breve* M-16V (Morinaga) is present per g powder. Fat is for the main part of vegetable origin but also tuna fish oil (source of DHA), algae oil (source of ARA) arachidonic acid (ARASCO, Martek) and egg lipid (source of DHA and ARA) are present as a source of LC-PUFA, resulting in 0.52 wt% ARA based on total fatty acyl chains and 0.40 wt% DHA based on total fatty acyl chains. Furthermore the composition comprises minerals, trace elements, vitamins, and other micronutrients as known in the art and according to guidelines for preterm infants. For a ready to drink formula, the instructions are to dilute 16.9 g powder (3 scoops) with water until a final volume of 100 ml.

### Example 4: Post discharge formula for use in premature or small for gestational infants after discharged from the hospital (compositions as such not falling under the scope of the present invention)

Post-discharge formula are marketed for use in premature or small for gestational infants after discharged from the hospital or after when reaching the corrected a term age, until a corrected age of 6 months. In powder form the formula comprises per 100 g about 491 kcal, 13.4 g protein, 49.1 g digestible carbohydrates (mainly lactose and maltodextrin), 26 g fat and 5.2 g non-digestible oligosaccharides. The protein comprises about 92.5 wt.% casein and whey protein from cow's milk based on total protein in a weight ratio of 1:1.5. About 7.5 wt.% of the protein is free L-glutamine. The non-digestible oligosaccharides are galacto-oligosaccharides (Source Vivinal GOS, Borculo Domo) and fructo-polysaccharides (Source RaftilinHP, Orafti ) in a weight ratio of 9:1. Due to the EU directives the non digestible disaccharides in the GOS do not qualify as dietary fiber. Hence the fiber content is labeled to be 3.7 g per 100 g powder. 5.10⁷ cfu of *Bifidobacterium breve* M-16V (Morinaga) is present per g powder. Fat is for the main part of vegetable origin but also fish oil, algae oil and egg lipid are present as a source of LC-PUFA are present, resulting in 0.44 wt.% ARA based on total fatty acyl chains and 0.33 wt.% DHA based on total fatty acyl chains. Furthermore the composition comprises minerals, trace elements, vitamins, L-carnitine, choline, my-inositol and taurine and nucleotides as known in the art and according to guidelines for preterm infants. For a ready to drink formula, the instructions are to dilute 15.3 g powder (3 scoops) with water until a final volume of 100 ml.

## Claims

1. A nutritional composition comprising a prebiotic fiber and a probiotic bacterium and glutamine for use in accelerating learning ability and/or accelerating reaching memory objectives in an infant, wherein the infant is a preterm infant and/or an infant that is born small for gestational age (SGA), and wherein the acceleration is in comparison to preterm infants and/or infants that are born small for gestational age (SGA) not being adminstered the nutritional composition comprising prebiotic fiber and probiotic bacterium and glutamine.

2. The nutritional composition for use according to claim 1, wherein the prebiotic fiber is selected from the group consisting of a fructo-oligosaccharide, a galacto-oligosaccharide, a fructo-polysaccharide, a combination of a fructo-oligosaccharide and a galacto-oligosaccharide, a combination of a fructo-oligosaccharide and a fructo-polysaccharide, a combination of a galacto-oligosaccharide and a fructo-polysaccharide and a combination of a fructo-oligosaccharide, a galacto-oligosaccharide and a fructo-polysaccharide.

3. The nutritional composition for use according to any one of the preceding claims, wherein the prebiotic fiber is present in an amount of 0.5 to 20 wt.% based on dry weight of the nutritional composition.

4. The nutritional composition for use according to any one of the preceding claims, wherein the probiotic bacterium is a *Bifidobacterium,* preferably a *Bifidobacterium breve.,* more preferably *Bifidobacterium breve* M 16V.

5. The nutritional composition for use according to any one of the preceding claims, wherein the daily dose of glutamine provided to the infant is between 0.01 and 0.5 g/kg body weight, preferably between 0.05 and 0.4 g/kg body weight, even more preferably between 0.1 and 0.35 g/kg body weight.

6. The nutritional composition for use according to any one of the preceding claims, wherein the probiotic bacterium is present in an amount of 10² to 10¹³ cfu per g dry weight of the nutritional composition.

7. The nutritional composition for use according to any one of the preceding claims, wherein the composition comprises glutamine in the form of free amino acid, glutamine containing dipeptide and/or glutamine containing tripeptide.

8. The nutritional composition for use according to claim 7, wherein the glutamine in the form of free amino acid, glutamine containing dipeptide and/or glutamine containing tripeptide is present in an amount of at least 1.5 wt.% based on dry weight of the nutritional composition.

9. The nutritional composition for use according to any one of the preceding claims, wherein the nutritional composition is an infant formula.

10. The nutritional composition for use according to any one of the preceding claims, wherein the nutritional composition is a preterm infant formula.

11. The nutritional composition for use according to any one of the preceding claims, wherein the infant is a mammalian infant, preferably a human infant, most preferably a human preterm infant.

12. The nutritional composition for use according to any one of the preceding claims, wherein the nutitional composition is for administraion in the first three months of life of the infant, preferably in the first month of the life of the infant.

13. The nutritional composition for use according to any one of the preceding claims wherein accelerating learning ability and/or accelerating reaching memory objectives in an infant occurs in the first 6 months of life, preferably in the first 3 months of life.

## Patentansprüche

1. Nährstoffzusammensetzung, umfassend einen prebiotischen Ballaststoff und ein probiotisches Bakterium und Glutamin zur Anwendung bei der Beschleunigung der Lernfähigkeit und/oder der Beschleunigung des Erreichens von Gedächtniszielen bei einem Säugling, wobei der Säugling ein Frühgeborenes und/oder ein Säugling ist, der für sein Gestationsalter (SGA) klein geboren wurde, und wobei die Beschleunigung im Vergleich zu Frühgeborenen und/oder Säuglingen, die für ihr Gestationsalter (SGA) klein geboren wurden, erfolgt, die nicht die Nährstoffzusammensetzung verabreicht bekommen, die einen prebiotischen Ballaststoff und ein probiotisches Bakterium und Glutamin umfasst.

2. Nährstoffzusammensetzung zur Anwendung nach Anspruch 1, wobei der prebiotische Ballaststoff aus der Gruppe ausgewählt ist, die aus einem Fructooligosaccharid, einem Galactooligosaccharid, einem Fructopolysaccharid, einer Kombination aus einem Fructooligosaccharid und einem Galactooligosaccharid, einer Kombination aus einem Fructooligosaccharid und einem Fructopolysaccharid, einer Kombination aus einem Galactooligosaccharid und einem Fructopolysaccharid und einer Kombination aus einem Fructooligosaccharid, einem Galactooligosaccharid und einem Fructopolysaccharid besteht.

3. Nährstoffzusammensetzung zur Anwendung nach einem der voranstehenden Ansprüche, wobei der prebiotische Ballaststoff in einer Menge von 0,5 bis 20 Gew.-%, bezogen auf das Trockengewicht der Nährstoffzusammensetzung, vorhanden ist.

4. Nährstoffzusammensetzung zur Anwendung nach einem der voranstehenden Ansprüche, wobei das probiotische Bakterium ein *Bifidobacterium,* vorzugsweise ein *Bifidobacterium breve,* besonders bevorzugt *Bifidobacterium breve* M 16V ist.

5. Nährstoffzusammensetzung zur Anwendung nach einem der voranstehenden Ansprüche, wobei die tägliche Dosis an Glutamin, die dem Säugling bereitgestellt wird, zwischen 0,01 und 0,5 g/kg Körpergewicht, vorzugsweise zwischen 0,05 und 0,4 g/kg Körpergewicht und noch bevorzugter zwischen 0,1 und 0,35 g/kg Körpergewicht beträgt.

6. Nährstoffzusammensetzung zur Anwendung nach einem der voranstehenden Ansprüche, wobei das probiotische Bakterium in einer Menge von 10² bis 10¹³ kbE pro g Trockengewicht der Nährstoffzusammensetzung vorhanden ist.

7. Nährstoffzusammensetzung zur Anwendung nach einem der voranstehenden Ansprüche, wobei die Zusammensetzung Glutamin in Form einer freien Aminosäure, eines Glutamin enthaltenden Dipeptids und/oder eines Glutamin enthaltenden Tripeptids umfasst.

8. Nährstoffzusammensetzung zur Anwendung nach Anspruch 7, wobei das Glutamin in Form einer freien Aminosäure, eines Glutamin enthaltenden Dipeptids und/oder eines Glutamin enthaltenden Tripeptids in einer Menge von mindestens 1,5 Gew.-%, bezogen auf das Trockengewicht der Nährstoffzusammensetzung, vorhanden ist.

9. Nährstoffzusammensetzung zur Anwendung nach einem der voranstehenden Ansprüche, wobei die Nährstoffzusammensetzung eine Säuglingsanfangsnahrung ist.

10. Nährstoffzusammensetzung zur Anwendung nach einem der voranstehenden Ansprüche, wobei die Nährstoffzusammensetzung eine Säuglingsanfangsnahrung für Frühgeborene ist.

11. Nährstoffzusammensetzung zur Anwendung nach einem der voranstehenden Ansprüche, wobei der Säugling ein Säugetier-Säugling, vorzugsweise ein menschlicher Säugling, am meisten bevorzugt ein menschlicher frühgeborener Säugling ist.

12. Nährstoffzusammensetzung zur Anwendung nach einem der voranstehenden Ansprüche, wobei die Nährstoffzusammensetzung zur Verabreichung in den ersten drei Lebensmonaten des Säuglings, vorzugsweise im ersten Lebensmonat des Säuglings, vorgesehen ist.

13. Nährstoffzusammensetzung zur Anwendung nach einem der voranstehenden Ansprüche, wobei das Beschleunigen der Lernfähigkeit und/oder das Beschleunigen des Erreichens von Gedächtniszielen bei einem Säugling in den ersten 6 Lebensmonaten, vorzugsweise in den ersten 3 Lebensmonaten, erfolgt.

## Revendications

1. Composition nutritionnelle comprenant une fibre prébiotique et une bactérie probiotique et de la glutamine pour utilisation dans l'accélération de la capacité d'apprentissage et/ou l'accélération de l'atteinte des objectifs de mémoire chez un nourrisson, dans laquelle le nourrisson est un nourrisson prématuré et/ou un nourrisson qui est né petit pour l'âge gestationnel (SGA), et dans laquelle l'accélération est en comparaison avec des nourrissons prématurés et/ou des nourrissons qui sont nés petits pour l'âge gestationnel (SGA) ne recevant pas la composition nutritionnelle comprenant une fibre prébiotique et une bactérie probiotique et de la glutamine.

2. Composition nutritionnelle pour utilisation selon la revendication 1, dans laquelle la fibre prébiotique est choisie dans le groupe consistant en un fructo-oligosaccharide, un galacto-oligosaccharide, un fructo-polysaccharide, une combinaison d'un fructo-oligosaccharide et d'un galacto-oligosaccharide, une combinaison d'un fructo-oligosaccharide et d'un fructo-polysaccharide, une combinaison d'un galacto-oligosaccharide et d'un fructo-polysaccharide et une combinaison d'un fructo-oligosaccharide, d'un galacto-oligosaccharide et d'un fructo-polysaccharide.

3. Composition nutritionnelle pour utilisation selon l'une quelconque des revendications précédentes, dans laquelle la fibre prébiotique est présente en une quantité de 0,5 à 20 % en poids sur la base du poids sec de la composition nutritionnelle.

4. Composition nutritionnelle pour utilisation selon l'une quelconque des revendications précédentes, dans laquelle la bactérie probiotique est un *Bifidobacterium,* de préférence un *Bifidobacterium breve,* de manière plus préférée *Bifidobacterium breve* M 16V.

5. Composition nutritionnelle pour utilisation selon l'une quelconque des revendications précédentes, dans laquelle la dose quotidienne de glutamine fournie au nourrisson est comprise entre 0,01 et 0,5 g/kg de poids corporel, de préférence entre 0,05 et 0,4 g/kg de poids corporel, de manière encore plus préférée entre 0,1 et 0,35 g/kg de poids corporel.

6. Composition nutritionnelle pour utilisation selon l'une quelconque des revendications précédentes, dans laquelle la bactérie probiotique est présente en une quantité de 10² à 10¹³ ufc par g de poids sec de la composition nutritionnelle.

7. Composition nutritionnelle pour utilisation selon l'une quelconque des revendications précédentes, dans laquelle la composition comprend de la glutamine sous forme d'acide aminé libre, de dipeptide contenant de la glutamine et/ou de tripeptide contenant de la glutamine.

8. Composition nutritionnelle pour utilisation selon la revendication 7, dans laquelle la glutamine sous la forme d'un acide aminé libre, d'un dipeptide contenant de la glutamine et/ou d'un tripeptide contenant de la glutamine est présente en une quantité d'au moins 1,5 % en poids sur la base du poids sec de la composition nutritionnelle.

9. Composition nutritionnelle pour utilisation selon l'une quelconque des revendications précédentes, dans laquelle la composition nutritionnelle est une préparation pour nourrisson.

10. Composition nutritionnelle pour utilisation selon l'une quelconque des revendications précédentes, dans laquelle la composition nutritionnelle est une préparation pour nourrison prématuré.

11. Composition nutritionnelle pour utilisation selon l'une quelconque des revendications précédentes, dans laquelle le nourrisson est un nourrisson mammifère, de préférence un nourrisson humain, de la manière la plus préférée un nourrisson prématuré humain.

12. Composition nutritionnelle pour utilisation selon l'une quelconque des revendications précédentes, dans laquelle la composition nutritionnelle est à administrer au cours des trois premiers mois de vie du nourrisson, de préférence au cours du premier mois de vie du nourrisson.

13. Composition nutritionnelle pour utilisation selon l'une quelconque des revendications précédentes, dans laquelle l'accélération de la capacité d'apprentissage et/ou l'accélération de l'atteinte des objectifs de mémoire chez un nourrisson se produit au cours des 6 premiers mois de vie, de préférence au cours des 3 premiers mois de vie.
